# EUROPÄISCHE PATENTANMELDUNG

(11) **EP 1 793 228 A1**
(43) Veröffentlichungstag der Anmeldung: **06.06.2007**
(21) Anmeldenummer: 05026525.5
(22) Anmeldetag: 05.12.2005
(51) Int. Cl.: G01N 33/487, A61B 5/00

(54) **Verfahren zum akustischen Ausgeben einer Information in einem Analysesystem**

(71) Anmelder: F. HOFFMANN-LA ROCHE AG, 4070 Basel (CH); Roche Diagnostics GmbH, 68305 Mannheim (DE)
(72) Erfinder: Werner, Karl, Wiesloch (DE); Albrecht, Gertrud, 68238 Mannheim (DE)
(74) Vertreter: Huhn, Michael

(57) **Zusammenfassung**

Die Erfindung bezieht sich auf ein Verfahren zum akustischen Ausgeben einer Information durch einen akustischen Signalgeber, wobei der Signalgeber in einem Analysesystem angeordnet ist und das Analysesystem eine Analyse einer flüssigen Probe mittels eines Testelements durchfuhrt, wobei Analyseergebnisse generiert und durch eine Datenverarbeitungseinheit verarbeitet werden und wobei eine Steuereinheit des Analysesystems den Signalgeber ansteuert, so dass der Signalgeber akustisch eine Information ausgibt, wobei der Signalgeber ein akustisches Prüfsignal abgibt, um den Signalgeber auf Funktionsfähigkeit zu prüfen.

## Beschreibung

Die Erfindung bezieht sich auf ein Verfahren zum akustischen Ausgeben einer Information durch einen akustischen Signalgeber, wobei der Signalgeber in einem Analysesystem zur Analyse einer Probe mittels eines Testelements angeordnet ist und sie bezieht sich auf ein Analysesystem mit einem akustischen Signalgeber.

Zur Analyse von Proben, beispielsweise Körperflüssigkeiten wie Blut oder Urin, werden häufig Analysesysteme verwendet, bei denen die zu analysierenden Proben auf ein Testelement gegeben werden und gegebenenfalls mit einer oder mehreren Reagenzien in einem Testfeld auf dem Testelement reagieren, bevor sie analysiert werden. Die optische, insbesondere photometrische, und die elektrochemische Auswertung von Testelementen stellen die gebräuchlichsten Verfahren zur schnellen Bestimmung der Konzentration von Analyten in Proben dar. Photometrische und elektrochemische Auswertungen werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Testelemente, die photometrisch oder elektrochemisch ausgewertet werden, einen großen Stellenwert.

In den letzten Jahren haben tragbare Messgeräte zur Blutzuckerbestimmung an Bedeutung gewonnen. Sie ermöglichen zu jeder Zeit mittels eines einfach zu bedienenden Messgeräts, einer hinsichtlich des Einstechschmerzes optimierten Stechhilfe und eines Testelements für den einmaligen Gebrauch, Blutzuckermesswerte zu bestimmen und damit eine genauere Insulindosierung des Patienten zur Stabilisierung seines Blutzuckerwertes vorzunehmen.

Es gibt verschiedene Formen von Testelementen. Bekannt sind z.B. im Wesentlichen quadratische Blättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Zur räumlichen Trennung von Detektionszone und Probenaufgabestelle eines Testelements sind im Stand der Technik kapillare Testelemente bekannt, z.B. aus WO 99/29429.

Die mittels eines Testelements in einem Analysesystem durchgeführte Analyse einer Probe führt zu einem Analyseergebnis, z.B. zu einem ermittelten Blutzuckerkonzentrationswert. Dieses Analyseergebnis wird üblicherweise auf einem optischen Anzeigefeld des Analysesystems, z.B. einer Flüssigkristallanzeige angezeigt. Für Anwender, die unter einer Sehbehinderung oder Blindheit leiden, wird zusätzlich eine akustische Ausgabe vorgesehen.

WO 03/039362 A1 bezieht sich auf ein reagenzloses Vollblut-Blutzuckermessgerät, bei dem ein Prozessor gemessene Konzentrationsergebnisse und/oder andere Informationen an ein Steuergerät kommuniziert. Das Steuergerät betreibt ein optisches Anzeigefeld zum Präsentieren der Information für einen Anwender. Zusätzlich oder alternativ zu dem optischen Anzeigefeld kann eine akustische Ausgabe der Information vorgesehen sein.

WO 02/062212 A1 betrifft ein Verwaltungssystem für das persönliche Befinden, das eine Ausgabevorrichtung umfasst, die einem Patienten eine Behandlungsempfehlung kommuniziert. Die Ausgabevorrichtung kann die Behandlungsempfehlung durch hörbare, sichtbare oder taktile Mittel übermitteln, beispielsweise durch einen Warntongeber oder eine Systemanzeige.

Zum akustischen Ausgeben einer Information dient üblicherweise ein akustischer Signalgeber oder ein Lautsprecher.

US 4,014,016 hat ein Ausgabesystem für Blinde zum Gegenstand, bei dem Informationen durch akustische Signale übermittelt werden. Dabei handelt es sich z.B. um ein Ausgabesystem eines Taschenrechners, bei dem die Ziffern eines Rechenergebnisses durch einheitlich beabstandete Tonfolgen mit einer unterschiedlichen Anzahl von Tönen derselben Frequenz augegeben werden.

DE 25 50 614 bezieht sich auf ein Verfahren zur hörbaren Ausgabe durch das Erzeugen eines codierten elektrischen Ausgangssignals, welches bezeichnend für eine wiedererlangbare Information ist. Dieses codierte elektrische Ausgangssignal wird zum Erzeugen einer vorbestimmten Anzahl von Hörtönen mit im Wesentlichen der gleichen Frequenz verwendet, wobei die Anzahl dieser Töne bezeichnend für die wiederzuerlangende Information ist. Die wiedererlangbare Information kann z.B. wenigstens eine Dezimalzahl sein, wobei die Anzahl der hörbaren Töne gleich der Dezimalzahl ist.

Eine akustische Ausgabe in einem Analysesystem zur Analyse einer Probe mittels eines Testelements muss besondere Anforderungen an seine Funktionsfähigkeit erfüllen. Von dem akustisch korrekt ausgegebenen Ergebnis der Analyse kann die Gesundheit oder auch das Leben des Anwenders abhängen. Beispielsweise hängt von einem gemessenen Blutglukosewert die Insulindosis ab, die einem Diabetiker verabreicht wird.

Daher ist es Aufgabe der vorliegenden Erfindung, akustische Fehlausgaben durch einen akustischen Signalgeber eines Analysesystems zur Analyse einer Probe mittels eines Testelements zu vermeiden beziehungsweise für den Anwender erkennbar zu machen.

Diese Aufgabe wird erfindungsgemäß gelöst durch ein Verfahren zum akustischen Ausgeben einer Information durch einen akustischen Signalgeber, wobei der Signalgeber in einem Analysesystem angeordnet ist und das Analysesystem eine Analyse einer flüssigen Probe mittels eines Testelements durchführt, wobei Analyseergebnisse generiert und durch eine Datenverarbeitungseinheit verarbeitet werden und wobei eine Steuereinheit des Analysesystems den Signalgeber ansteuert, so dass der Signalgeber akustisch eine Information ausgibt, wobei der Signalgeber ein akustisches Prüfsignal abgibt, um den Signalgeber auf Funktionsfähigkeit zu prüfen.

Ein Analysesystem ist in diesem Zusammenhang ein System zur Analyse einer flüssigen Probe, beispielsweise zur Analyse von Blut, Urin oder interstitieller Flüssigkeit, insbesondere ein Blutzuckermessgerät. Die Datenverarbeitungseinheit des Analysesystems empfängt die bei der Analyse einer auf dem Testelement angeordneten flüssigen Probe generierten Analyseergebnisse und verarbeitet diese. Beispielsweise berechnet sie aus den (z.B. photometrisch oder elektrochemisch) gemessenen Analyseergebnissen die Konzentration eines Analyten in der flüssigen Probe. Die in dem Analysesystem enthaltene Steuereinheit steuert u.a. den Signalgeber an, z.B. wenn über den Signalgeber die Information ausgegeben werden soll, welches durch die Datenverarbeitungseinheit verarbeitete Analyseergebnis sich aus der Analyse einer flüssigen Probe ergeben hat.

Als akustischer Signalgeber des Analysesystems kann jeder dem Fachmann geläufige akustische Signalgeber vorliegen, z.B. ein Summer, ein Lautsprecher oder ein piezoelektrischer Signalgeber.

Die ausgegebene Information kann z.B. eine Information zu dem Betriebsstatus des Analysesystems oder zu einem Analyseergebnis sein.

Erfindungsgemäß gibt der Signalgeber in dem Analysesystem ein akustisches Prüfsignal ab, um die Funktionsfähigkeit des Signalgebers zu prüfen. Der Anwender der Analysesystems kann dann erkennen, ob das akustische Prüfsignal korrekt ist oder nicht. Falls das akustische Prüfsignal von dem Signalgeber nicht korrekt abgegeben wird, ist davon auszugehen, dass eine durch den akustischen Signalgeber ausgegebene Information ebenfalls inkorrekt sein könnte.

Die Erfindung bezieht sich weiterhin auf ein Analysesystem zur Analyse einer flüssigen Probe mittels eines Testelements, enthaltend einen akustischen Signalgeber zum akustischen Ausgeben einer Information, ein Analysegerät zur Generierung von Analyseergebnissen, eine Datenverarbeitungseinheit zum Verarbeiten der Analyseergebnisse und eine Steuereinheit, wobei die Steuereinheit so ausgelegt ist, dass sie den Signalgeber zum Abgeben eines akustischen Prüfsignals ansteuert, um den Signalgeber auf Funktionsfähigkeit zu prüfen.

Gemäß einer bevorzugten Ausführungsform des erfindungsgemäßen Analysesystems enthält das Analysesystem einen Schalter, der durch das Einschieben oder Einlegen eines Testelements in das Analysesystem ausgelöst wird, wobei der Schalter so an das Steuergerät gekoppelt ist, dass das Steuergerät beim Auslösen des Schalters den Signalgeber zum Abgeben eines akustischen Prüfsignals ansteuert. Ein Testelement muss dabei manuell oder automatisch vor der Durchführung einer Analyse eine flüssigen Probe in eine Probenaufnahmeposition zur Aufnahme der flüssigen Probe auf das Testelement und/oder eine Messposition zur Durchführung einer Messung und Generierung von Analyseergebnissen in das Analysesystem eingeschoben oder eingelegt werden.

Das akustische Prüfsignal kann aber auch abgegeben werden, sobald der Benutzer des Analysesystems einen bestimmten Knopf des Analysesystems drückt (z.B. den Knopf zum Einschalten des Analysesystems) oder automatisch zu mindestens einem bestimmten Zeitpunkt während des Betriebes des Analysesystems.

Gemäß einer bevorzugten Ausführungsform der vorliegenden Erfindung gibt der Signalgeber ein akustisches Prüfsignal ab, das einen Ton oder eine Folge von Tönen umfasst, wobei der Ton oder die Folge von Tönen im Wesentlichen eine gleich bleibende Frequenz aufweist oder wobei der Ton oder die Folge von Tönen eine sich ändernde Frequenz aufweist.

Die Folge von Tönen kann dabei so abgegeben werden, dass die einzelnen Töne ineinander übergehen oder zumindest teilweise durch Pausen zwischen den Tönen unterbrochen werden.

Ferner kann der Signalgeber bei dem erfindungsgemäßen Verfahren ein akustisches Prüfsignal abgeben, das eine Folge von Tönen umfasst, wobei die Töne zumindest teilweise verschiedene Tonlängen aufweisen oder wobei die Töne im Wesentlichen die gleiche Tonlänge aufweisen.

Der akustische Signalgeber kann erfindungsgemäß auch ein akustisches Prüfsignal abgeben, das einen Ton oder eine Folge von Tönen umfasst, wobei sich die Lautstärke des Tons oder der Folge von Tönen ändert oder wobei der Ton oder die Folge von Tönen im Wesentlichen die gleiche Lautstärke aufweist.

Das akustische Prüfsignal sollte insbesondere hinsichtlich Tonfrequenzen, Tonlängen, Tonlautstärken, Frequenz- und Lautstärkeänderungen möglichst alle Merkmale aufweisen, die ein von dem Signalgeber im normalen Betrieb des Analysesystems abgegebenes akustisches Signal zum Ausgeben einer Information aufweisen kann. Falls ein solches akustisches Signal zum Ausgeben einer Information lediglich aus einer Folge von Tönen im Wesentlichen derselben Frequenz, Tonlänge und Lautstärke besteht, wobei die einzelnen Töne durch Pausen unterbrochen werden, so sollte das Prüfsignal zumindest dieselben Merkmale aufweisen, um eine Fehlfunktion sicher feststellen zu können. Ferner sollte das Prüfsignal möglichst so gestaltet werden, dass ein Anwender das korrekte Prüfsignal und eventuelle Abweichungen davon leicht erkennen kann.

Gemäß einer bevorzugten Ausführungsform der vorliegenden Erfindung gibt der Signalgeber das Prüfsignal zu Beginn einer Inbetriebnahme des Analysesystems ab. Der Signalgeber kann aber auch zu jedem anderen Zeitpunkt des Betriebs des Analysesystems das oder mindestens ein zusätzliches Prüfsignal abgeben. Der Vorteil einer Abgabe des Prüfsignals zu Beginn einer Inbetriebnahme des Analysesystems ist, dass der Anwender von der Durchführung einer Analyse absehen kann, wenn er gleich zu Beginn eine Fehlfunktion des akustischen Signalgebers des Analysesystems feststellt. Damit können z.B. nutzlose schmerzhafte Blutentnahmen oder eine Verschwendung von Testelementen für Analysen mit dem Analysesystem vermieden werden, die aufgrund der Fehlfunktion des akustischen Signalgebers nicht verwertbar sind. Der Anwender kann bei Erkennen eines fehlerhaften Prüfsignals aber auch einen nicht sehbehinderten Betreuer um ein Ablesen der Information auf einer optischen Anzeige bitten, falls eine solche optische Anzeige vorhanden ist.

Die Abgabe des Prüfsignals gleich zu Beginn der Inbetriebnahme des Analysesystems kann beispielsweise durch das Einschalten des Analysesystems oder durch das Einschieben eines Testelements automatisch oder durch den Anwender in das Analysesystem ausgelöst werden. Die Überprüfung der Funktionsfähigkeit des Signalgebers mittels des akustischen Prüfsignals kann gleichzeitig mit der Überprüfung der Funktionsfähigkeit einer optischen Anzeige des Analysesystems, z.B. einer Flüssigkristallanzeige, erfolgen, so dass keine zusätzliche Verzögerung bis zur Einsatzbereitschaft des Analysesystems in Kauf genommen werden muss.

Gemäß einer bevorzugten Ausführungsform der vorliegenden Erfindung codiert der akustische Signalgeber eine Information akustisch, die als Zahl mit mindestens einer Ziffer darstellbar ist. Die Zahl kann z.B. eine mit dem Analysesystem ermittelte Konzentration eines Analyten in einer auf einem Testelement analysierten Probe sein, insbesondere die Glukosekonzentration in Blut. Die Zahl kann aus einer oder mehreren Ziffern bestehen und gegebenenfalls Nachkommastellen aufweisen.

Zusätzlich oder alternativ dazu kann die durch den akustischen Signalgeber codierte Information eine Information bezüglich der Betriebsbereitschaft des Analysesystems (z.B. dass das Analysesystem eingeschaltet wurde) oder eine Information über den Zeitpunkt zur Durchführung einer bestimmten Handlung durch den Anwender (z.B. den Zeitpunkt zum Anlegen seines Fingers an eine in das Analysesystem integrierte Stechhilfe oder den Zeitpunkt zum Übertragen einer zu analysierenden Probe auf ein Testelement) umfassen. Der Signalgeber kann auch akustisch eine Information bezüglich der Notwendigkeit zum Auswechseln oder Nachfüllen eines in dem Analysesystem verwendeten Verbrauchsmittels (z.B. Testelement, Testelementmagazin, Batterie, Lanzette oder Disposable) ausgeben.

Gemäß einer bevorzugten Ausführungsform der vorliegenden Erfindung codiert der akustische Signalgeber eine Information als Folge von Tönen akustisch, wobei die Anzahl der Töne bezeichnend für die Information ist. Eine Zahl kann beispielsweise so akustisch ausgegeben werden, dass eine Anzahl von hörbaren Ausgangstönen jeweils einem Ziffernwert für jede Ziffer der Zahl entspricht, wobei die Ausgabe jeweils einer neuen Ziffer z.B. durch eine längere Pause oder ein anderes akustisches Signal ausgegeben wird. Ferner kann ein bestimmtes akustisches Signal für einen Dezimalpunkt und/oder ein Vorzeichen vorgesehen sein.

Des Weiteren bezieht sich die Erfindung auf die Verwendung eines akustischen Signalgebers, der zum akustischen Ausgeben einer Information in einem Analysesystem zur Analyse einer Probe mittels eines Testelements angeordnet ist, zur Überprüfung der Funktionsfähigkeit des Signalgebers durch eine Abgabe eines akustischen Prüfsignals.

## Patentansprüche

1. Verfahren zum akustischen Ausgeben einer Information durch einen akustischen Signalgeber, wobei der Signalgeber in einem Analysesystem angeordnet ist und das Analysesystem eine Analyse einer flüssigen Probe mittels eines Testelements durchführt, wobei Analyseergebnisse generiert und durch eine Datenverarbeitungseinheit verarbeitet werden und wobei eine Steuereinheit des Analysesystems den Signalgeber ansteuert, so dass der Signalgeber akustisch eine Information ausgibt, **dadurch gekennzeichnet, dass** der Signalgeber ein akustisches Prüfsignal abgibt, um den Signalgeber auf Funktionsfähigkeit zu prüfen.

2. Verfahren gemäß Anspruch 1, **dadurch gekennzeichnet, dass** der Signalgeber ein akustisches Prüfsignal abgibt, das einen Ton oder eine Folge von Tönen umfasst, wobei der Ton oder die Folge von Tönen im Wesentlichen eine gleich bleibende Frequenz aufweist oder wobei der Ton oder die Folge von Tönen eine sich ändernde Frequenz aufweist.

3. Verfahren gemäß einem der Ansprüche 1 oder 2, **dadurch gekennzeichnet, dass** der Signalgeber ein akustisches Prüfsignal abgibt, das eine Folge von Tönen umfasst, wobei die Töne zumindest teilweise verschiedene Tonlängen aufweisen oder wobei die Töne im Wesentlichen die gleiche Tonlänge aufweisen.

4. Verfahren gemäß einem der Ansprüche 1 bis 3, **dadurch gekennzeichnet, dass** der Signalgeber ein akustisches Prüfsignal abgibt, das einen Ton oder eine Folge von Tönen umfasst, wobei sich die Lautstärke des Tons oder der Folge von Tönen ändert oder wobei der Ton oder die Folge von Tönen im Wesentlichen die gleiche Lautstärke aufweist.

5. Verfahren gemäß einem der Ansprüche 1 bis 4, **dadurch gekennzeichnet, dass** der Signalgeber das Prüfsignal zu Beginn einer Inbetriebnahme des Analysesystems abgibt.

6. Verfahren gemäß einem der Ansprüche 1 bis 5, **dadurch gekennzeichnet, dass** der akustische Signalgeber eine Information akustisch codiert, die als Zahl mit mindestens einer Ziffer darstellbar ist.

7. Verfahren gemäß einem der Ansprüche 1 bis 6, **dadurch gekennzeichnet, dass** der akustische Signalgeber eine Information als Folge von Tönen akustisch codiert, wobei die Anzahl der Töne bezeichnend für die Information ist.

8. Analysesystem zur Analyse einer flüssigen Probe mittels eines Testelements, enthaltend einen akustischen Signalgeber zum akustischen Ausgeben einer Information, ein Analysegerät zur Generierung von Analyseergebnissen, eine Datenverarbeitungseinheit zum Verarbeiten der Analyseergebnisse und eine Steuereinheit, **dadurch gekennzeichnet, dass** die Steuereinheit so ausgelegt ist, dass sie den Signalgeber zum Abgeben eines akustischen Prüfsignals ansteuert, um den Signalgeber auf Funktionsfähigkeit zu prüfen.

9. Analysesystem gemäß Anspruch 8, **dadurch gekennzeichnet, dass** das Analysesystem einen Schalter enthält, der durch das Einschieben eines Testelements in das Analysesystem ausgelöst wird, wobei der Schalter so an das Steuergerät gekoppelt ist, dass das Steuergerät beim Auslösen des Schalters den Signalgeber zum Abgeben eines akustischen Prüfsignals ansteuert.

10. Verwendung eines akustischen Signalgebers, der zum akustischen Ausgeben einer Information in einem Analysesystem zur Analyse einer Probe mittels eines Testelements angeordnet ist, zur Überprüfung der Funktionsfähigkeit des Signalgebers durch eine Abgabe eines akustischen Prüfsignals.
